(19) Europäisches Patentamt
European Patent Office
Office européen des brevets

(11) Veröffentlichungsnummer: **0 237 912**
**A1**

(12) **EUROPÄISCHE PATENTANMELDUNG**

(21) Anmeldenummer: **87103384.1**

(22) Anmeldetag: **10.03.87**

(51) Int. Cl.⁴: **C07D 233/54**, C07D 249/08, C07D 249/16, C07D 257/04, C07D 401/12, A01N 43/50, A01N 43/653, A01N 43/713

(30) Priorität: **20.03.86 JP 60642/86**

(43) Veröffentlichungstag der Anmeldung:
**23.09.87 Patentblatt 87/39**

(84) Benannte Vertragsstaaten:
**AT BE CH DE ES FR GB GR IT LI NL SE**

(71) Anmelder: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Saito, Junichi**
**3-7-12 Osawa**
**Mitaka-shi Tokyo(JP)**
Erfinder: **Tamura, Tatsuo**
**1-7-30, Hanenaka, Hamura-machi**
**Nishitamagun Tokyo(JP)**
Erfinder: **Kurahashi, Yoshio**
**47-15, Oya-machi**
**Hachioji-shi Tokyo(JP)**
Erfinder: **Matsumoto, Noboru**
**39-15, Namiki-cho**
**Hachioji-shi Tokyo(JP)**
Erfinder: **Yamaguchi, Naoko**
**4-6-7-Shinmei**
**Hino-shi Tokyo(JP)**

(74) Vertreter: **Schumacher, Günter, Dr. et al**
**c/o Bayer AG Konzernverwaltung RP Patentabteilung**
**D-5090 Leverkusen 1 Bayerwerk(DE)**

(54) **Neue Sulfonylazole.**

(57) Sulfonylazole der Formel (I)

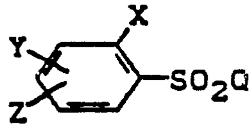

(I)

in der
X Wasserstoff, Alkyl, Halogen, Alkoxy oder Aryl bezeichnet,
Y Wasserstoff, Alkyl, Halogen, Nitro oder Alkoxy bezeichnet,
Z Wasserstoff, Alkyl, Halogen oder Alkoxy bezeichnet oder
Y und Z zusammen einen gegebenenfalls substituierten cyclischen Ring bezeichnen, der Hetero-Atome enthalten kann, wurden gefunden und ihre Wirkung als Fungizide im Pflanzenschutz.

**EP 0 237 912 A1**

Die neuen Sulfonylazole der FormeL (I) können aus geeigneten substituierten Phenylsulfonylhalogeniden mit geeigneten Heterocyclen in Gegenwart eines inerten Lösungsmittels hergestellt werden.

## Neue Sulfonylazole

Die vorliegende Erfindung betrifft neue Sulfonylazole, ein Verfahren zu ihrer Herstellung und ihre Verwendung als Fungizide in Landwritschaft und Gartenbau.

Es wurde bereits offenbart, daß bestimmte Sulfonylazole als Bleichmittel (siehe die JP-OS 64181/1979) und als landwirtschaftliches Fungizid (siehe die JP-OS 28053/1982) nützlich sind.

Nunmehr wurde gefunden, daß die Sulfonylazole der Formel (I)

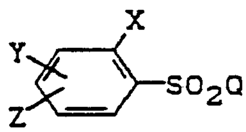

(I)

,

in der

X Wasserstoff, Alkyl, Halogen, Alkoxy oder Aryl bezeichnet,

Y Wasserstoff, Alkyl, Halogen, Nitro oder Alkoxy bezeichnet,

Z Wasserstoff, Alkyl, Halogen oder Alkoxy bezeichnet oder

Y und Z zusammen einen gegebenenfalls substituierten cyclischen Ring bezeichnen, der Hetero-Atome, zum Beispiel Sauerstoff, enthalten kann,

Q einen Rest der Formel

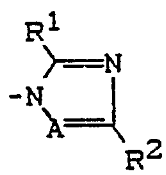

bezeichnet, in der

A einen Rest -N= oder einen Rest

$-\underset{R^3}{\overset{|}{C}}=$ bezeichnet,

und worin

$R^1$ Wasserstoff, Alkyl oder Aryl bezeichnet und

$R^2$ und $R^3$ jeweils Wasserstoff, Alkyl, Halogen oder Nitro bezeichenen,

mit der Maßgabe, daß nur in dem Fall, in dem Y und Z einen gegebenenfalls substituierten Ring bezeichnen, sämtliche R-Substituenten gleichzeitig Wasserstoff sein können, oder

Q einen 1H-Benzotriazol-1-yl-Rest oder 1H-Tetrazol-1-yl-Rest bezeichnet.

Die Verbindungen der Formel (I) können mit Hilfe eines allgemeinen Verfahrens hergestellt werden, bei dem eine Verbindung der Formel (II)

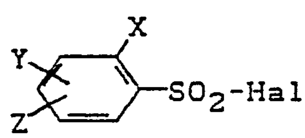

(II)

,

in der X, Y und Z die oben angegbenen Bedeutungen haben und Hal Halogen bezeichnet, mit einer Verbindung der Formel (III)

M -Q (III)

in der

Q die oben angegebenen Bedeutungen hat,

M Wasserstoff, ein Alkalimetall oder ein Erdalkalimetall-Äquivalent bezeichnet,

in Gegenwart eines inerten Lösungsmittels und gegebenenfalls in Gegenwart einer Base umgesetzt wird.

Die neuen Sulfonylazole der Formel (I) zeigen potente Wirkung als Fungizide in Landwirtschaft und Gartenbau und weisen ein breites Wirkungsspektrum auf, während sie Nutzpflanzen nicht schädigen. Insbesondere zeigen die aktiven Verbindungen der Formel (I) eine sehr hohe fungizide Wirkung gegen Braunfäule der Tomatenpflanzen und dergleichen

In der Formel (I) der erfindungsgemäßen Verbindungen bezeichnen

X vorzugsweise Alkyl mit 1 bis 4 Kohlenstoff-Atomen,

Y vorzugsweise Fluor, Chlor, Brom, Nitro oder Alkyl mit 1 bis 4 Kohlenstoff-Atomen,

Z vorzugsweise Wasserstoff und

Q vorzugsweise einen Rest der Formel

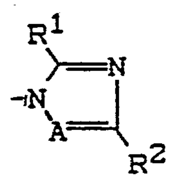

,

in der

A einen Rest

$-\underset{\underset{R^3}{|}}{C}=$ bezeichnet,

$R^1$ Wasserstoff oder Alkyl mit 1 bis 4 Kohlenstoff-Atomen bezeichnet und

$R^2$ und $R^3$ jeweils Wasserstoff oder Halogen bezeichnen,

mit der Maßgabe, daß nicht sämtliche R-Substituenten gleichzeitig Wasserstoff sind, oder

Q einen 1H-Benzotriazol-1-yl-Rest oder 1H-Tetrazol-1-yl-Rest bezeichnet.

Besonders bevorzugt bedeuten in der Formel (I)

X Methyl,

Y Chlor, Brom, Nitro oder Alkyl mit 1 bis 3 Kohlenstoff-Atomen,

Z Wasserstoff und

Q einen 2-Methylimidazol-1-yl-Rest, einen 4,5-Dichloroimidazol-1-yl-Rest, einen 1H-Benzotriazol-1-yl-Rest oder einen 1H-Tetrazol-1-yl-Rest.

Beispiele für Verbindungen der Formel (I) gemäß der Erfindung sind

1-(2-Chloro-6-methylbenzolsulfonyl)-2-methylimidazol,

1-(2,5-Dimethylbenzolsulfonyl)-1H-tetrazol,

1-(2-Methyl-5-nitrobenzolsulfonyl)-4,5-dichloroimidazol und

1-(5-Chloro-2-methyl-benzolsulfonyl)-1H-benzotriazol.

Wenn in dem Verfahren zur Herstellung der vorliegenden Verbindungen der Formel (I) 2-Methyl-5-nitrobenzolsulfonylchlorid und 4,5-Dichloroimidazol als Ausgangsstoffe eingesetzt werden, läßt sich der Reaktionsablauf durch die folgende Gleichung darstellen:

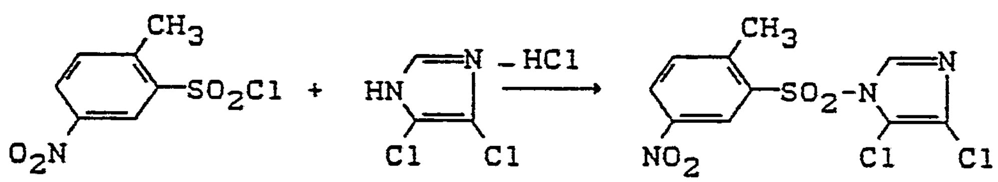


In der Formel (II) der Ausgangsverbindungen haven X, Y, Z und Hal die gleichen Bedeutungen wie im Fall der Verbindungen der Formel (I), die hergestellt werden sollen.

Weiterhin sind die bevorzugten Bedeutungen von X, Y und Z der Formel (II) die gleichen wie diejenigen, die für den Fall der Formel (I) angegeben sind. Hal bezeichnet vorzugsweise Chlor.

Die Verbindungen der Formel (II) sind im allgemeinen wohlbekannte Verbindungen der organischen Chemie. Beispiele für die Verbindungen der Formel (II) sind

2,5-Dimethylbenzolsulfonylchlorid,

2,6-Dimethylbenzolsulfonylchlorid,

5-Chloro-2-methylbenzolsulfonylchlorid,

5-Bromo-2-methylbenzolsulfonylchlorid,

2-Chloro-6-methylbenzolsulfonylchlorid,

2-Methyl-5-nitrobenzolsulfonylchlorid,

4-Bromo-2-methylbenzolsulfonylchlorid und

2-Chloro-5-tert-butylbenzolsulfonylchlorid.

In den Verbindungen der Formel (III) haben Q und M die gleichen Bedeutungen, wie sie in den vorhergehenden Absätzen angegeben sind. Die bevorzugte Bedeutung von Q ist die gleiche, wie diejenige, die für den Fall der Formel (I) angegeben ist. M bezeichnet vorzugsweise Wasserstoff oder ein Natrium-Äquivalent.

Die Verbindungen der Formel (III) sind im allgemeinen wohlbekannte Verbindungen in der organischen Chemie. Beispiele für die Verbindungen der Formel (III) sind Imidazol, 2-Methylimidazol, 2-Ethylimidazol, 4-Methylimidazol, 4,5-Dichloroimidazol, 1H-Tetrazol, 1H-Benzotriazol und dergleichen.

Was das oben bezeichnete 4,5-Dichloroimidazol betrifft, so kann diese Verbindung mittels eines bekannten, in der US-PS 3 409 606 offenbarten Verfahrens hergestellt werden.

Bevorzugte Verdünnungsmittel für das Verfahren zur Herstellung der Verbindungen der Formel (I) sind inerte organische Lösungsmittel. Beispiele für die Lösungsmittel sind Wasser; aliphatische, cycloaliphatische oder aromatische Kohlenwasserstoffe und chlorierte Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen, Chlorbenzol; Ether wie Diethylether, Methylethylether, Di-isopropylether, Dibutylether, Propylenoxid, Dioxan, Tetrahydrofuran; Ketone wie Aceton, Methylethylketon, Methylisopropylketon, Methylisobutylketon; Nitrile wie Acetonitril, Propionitril, Acrylnitril; Alkohole wie Methanol, Ethanol, Isopropanol, Butanol, Ethylenglycol; Ester wie Ethylacetat, Amylacetat; Säureamide wie Dimethylformamid, Dimethylacetamid; Sulfone und Sulfoxide wie Dimethylsulfoxid, Sulfolan; Basen wie Pyridin.

Die erfindungsgemäße Reaktion kann in Gegenwart eines säurebindenden Mittels durchgeführt werden. Beispiele für säurebindende Mittel sind übliche Alkylimetallhydroxide, -carbonate, -hydrogencarbonate und -alkoholate und tertiäre Amine wie Triethylamin, Diethylanilin, Pyridin etc..

Die Reaktionstemperatur bei dem erfindungsgemäßen Verfahren kann innerhalb eines weiten Bereichs variiert werden. Beispielsweise kann das Verfahren bei einer Temperatur von etwa -20 °C bis zum Siedepunkt der Reaktionsmischung, vorzugsweise bei einer Temperatur von etwa 0°C bis 100°C, durchgeführt werden.

Obwohl die Reaktion gemäß der Erfindung mit Vorteil unter normalem Druck durchgeführt wird, kann sie auch unter erhöhtem oder vermindertem Druck durchgeführt werden.

Bei der Durchführung des erfindungsgemäßen Verfahrens können zur Herstellung der angestrebten Verbindungen der Formel (I) die Verbindungen der Formel (III) in einer Menge von etwa 1 bis 1,2 mol auf 1 mol der Verbindungen der Formel (II) in Gegenwart einer Base wie Pyridin eingesetzt werden.

Die aktiven Verbindungen (I) gemäß der Erfindung zeigen eine potente mikrobizide Wirkung und können in der Praxis zur Bekämpfung unerwünschter Mikroorganismen eingesetzt werden. Die aktiven Verbindungen sind zur Verwendung als Pflanzenschutzmittel geeignet.

Fungizide Mittel werden im Pflanzenschutz beispielsweise eingesetzt zur Bekämpfung von
Plasmodiophoromycetes,
Oomycetes,
Chytridiomycetes,
Zygomycetes,
Ascomycetes,
Basidiomycetes und
Deuteromycetes,

Bakterizide Mittel werden im Pflanzenschutz beispielsweise eingesetzt zur Bekämpfung von
Pseudomonadaceae,
Rhizobiaceae,
Enterobacteriaceae,
Corynebacteriaceae und
Streptomycetaceae.

Einige der Organismen, die pilzliche und bakterielle Erkrankungen verursachen und unter die oben aufgeführten Sammelbegriffe fallen, seien im Folgenden als nichtbeschränkende Beispiele genannt:
Species Xanthomonas
wie beispielsweise Xanthomonas campestris pv. oryzae;
Species Pseudomonas
wie beispielsweise Pseudomonas syringae pv. Lachrymans;
Species Erwinia
wie beispielsweise Erwinia amylovora;
Species Pythium
wie beispielsweise Pythium ultimum;
Species Phytophthora

wie beispielsweise Phytophthora infestans;
Species Pseudoperonospora
wie beispielsweise Pseudoperonospora cubensis;
Species Plasmopara
wie beispielsweise Plasmopara viticola;
Species Peronospora
wie beispielsweise Peronospora pisi oder P. brassicae;
Species Erysiphe
wie beispielsweise Erysiphe graminis;
Species Sphaerotheca
wie beispielsweise Sphaerotheca fuliginea;
Species Podosphaera
wie beispielsweise Podosphaera leucotricha;
Species Venturia
wie beispielsweise Venturia inaequalis;
Species Pyrenophora
wie beispielsweise Pyrenophora teres oder P. graminea (Conidiale Form: Drechslera, Synonym: Helminthosporium);
Species Cochliobolus
wie beispielsweise Cochliobolus sativus (Conidiale Form: Drechslera, Synonym: Helminthosporium);
Species Uromyces
wie beispielsweise Uromyces appendiculatus;
Species Puccinia
wie beispielsweise Puccinia recondita;
Species Tilletia
wie beispielsweise Tilletia caries;
Species Ustilago
wie beispielsweise Ustilago nuda oder Ustilago avenae;
Species Pellicularia
wie beispielsweise Pellicularia sasakii;
Species Pyricularia
wie beispielsweise Pyricularia oryzae;
Species Fusarium
wie beispielsweise Fusarium culmorum;;
Species Botrytis
wie beispielsweise Botrytis cinerea;
Species Septoria
wei beispielsweise Septoria nodorum;
Species Leptosphaeria
wie beispielsweise Leptosphaeria nodorum;
Species Cercospora
wie beispielsweise Cercospora canescens;
Species Alternaria
wie beispielsweise Alternaria brassicae;
Species Pseudocercosporella
wie beispielsweise Pseudocercosporella herpotrichoides.

Die gute Tolerierung der aktiven Verbindungen durch die Pflanzen bei den zur Bekämpfung der Pflanzenerkrankungen erforderlichen Konzentrationen erlaubt die Behandlung der oberirdischen Pflanzenteile, der Teile der vegetativen Fortpflanzung und der Samen sowie des Bodens.

Insbesondere seien gemäß der vorliegenden Erfindung neben der bereits oben erwähnten Braunfäule der Tomate (Phytophthora infestans) die Brusone-Krankheit bei Reis (Pyricularia oryzae), der falsche Mehltau der Gurke, Melone und dergleichen (Pseudoperonospora cubensis) und der falsche Mehltau des Weinstocks (Plasmopara viticola) als Beispiele für die Pflanzenerkrankungen erwähnt.

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche und synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten, gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lössungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe und ebenso auch Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischen Materialien wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoff, Azo-Farbstoff, oder Metallphthalocyanin-Farbstoff, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-%, der aktiven Verbindung.

Die aktiven Verbindungen gemäß der vorliegenden Erfindung können in ihren Formulierungen oder ihren verschiedenen Anwendungsformen als Gemische mit anderen bekannten aktiven Verbindungen vorliegen, etwa mit Fungiziden, Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die aktiven Verbindungen können als solche eingesetzt werden, oder sie können in Form solcher Formulierungen oder Anwendungsformen, die daraus durch weitere Verdünnung hergestellt werden, etwa gebrauchsfertigen Lösungen, Emulsionen, Suspensionen, Pulvern, Pasten und Granulat, eingesetzt werden. Sie werden in üblicher Weise ausgebracht, etwa durch Bewässern, Eintauchen, Spritzen, Zerstäuben, Vernebeln, Verkampfen, Einspritzen, Bilden von Aufschlämmungen, Aufpinseln, Stäuben, Streuen, Trockenbedecken, Feuchtbedecken, Naßbedecken, Schlammbedecken und Umhüllen mit einer Kruste.

Bei der Behandlung von Pflanzenteilen können die Konzentrationen der aktiven Verbindung in den Anwendungsformen innerhalb eines beträchtlichen Bereichs variiert werden. Sie liegen im allgemeinen in einem Bereich von 1 bis 0,0001 Gew.-%, und vorzugsweise von 0,5 bis 0,001 Gew.-%.

Im Fall der Saatgut-Behandlung werden Mengen der aktiven Verbindung von beispielsweise 0,001 bis 50 g, vorzugsweise 0,01 bis 10 g, auf 1 kg des Saatguts zur Anwendung gebracht.

Zur Behandlung des Bodens werden im allgemeinen am Wirkungsort Konzentrationen der aktiven Verbindung von 0,00001 bis 0,1 Gew.-%, insbesondere 0,0001 bis etwa 0,02 Gew.-%, eingesetzt.

Die Erfindung wird durch die folgenden Beispiele näher erläutert. Es ist jedoch ausdrücklich anzumerken, daß der Umfang der Erfindung nicht auf die Beispiele beschränkt ist.

Herstellungsbeispiele

Beispiel 1

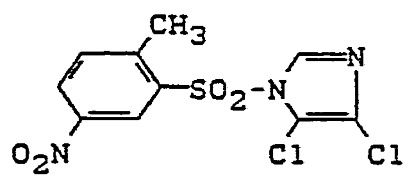

(Verbindung Nr. 22)

2,36 g 2-Methyl-5-nitrobenzolsulfonylchlorid und 2,74 g 4,5-Dichloroimidazol werden in 20 ml Pyridin gelöst, und die erhaltene Lösung wird 2 h bei 50°C gerührt. Die Reaktionslösung wird in 100 ml Eiswasser gegossen. Das Produkt, das auskristallisiert, wird mit einer kleinen Menge Diethylether gewaschen. 1,2 g 1-(2-Methyl-5-nitrobenzolsulfonyl)-4,5-dichloroimidazol mit einem Schmp. 125-130°C werden erhalten.

Beispiel 2

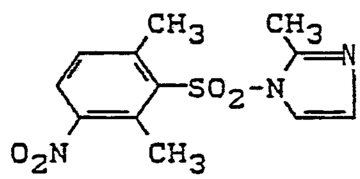

(Verbindung Nr. 15)

2,5 g 2,6-Dimethyl-3-nitrobenzolsulfonylchlorid und 1,64 g 2-Methylimidazol werden in 20 ml Pyridin gelöst, und die erhaltene Lösung wird 2 h bei 50°C gerührt. Die Reaktionslösung wird in 100 ml Eiswasser gegossen, um das Produkt auszukristallisieren. Das so erhaltene kristalline Produkt wird mit einer kleinen Menge Diethylether gewaschen. 1,5 g 1-(2,6-Dimethyl-3-nitrobenzolsulfonyl)-2-methylimidazol mit einem Schmp. 99-101°C werden erhalten.

Das als Ausgangsstoff in Beispiel 2 eingesetzte 2,6-Dimethyl-3-nitrobenzolsulfonylchlorid wird nach einem bekannten, in Helv. Chim. Acta 61 (8), 3079-3086, beschriebenen Verfahren hergestellt.

Die in gleicher Weise wie nach den obigen Beispielen erhältlichen Verbindungen der Formel (I) sind zusammen mit den Beispielen 1 und 2 in der folgenden Tabelle aufgeführt.

## Tabelle

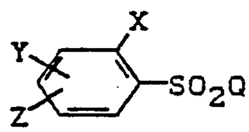

| Verbindung Nr. | X, Y, Z | Q | Physikalische Konstante |
|---|---|---|---|
| 1 | 2 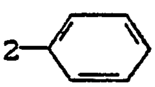 | 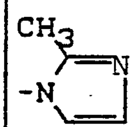 | Schmp. 77-79° C |
| 2 | 2,5-$(CH_3)_2$ | " | Schmp. 58-60° C |
| 3 | 2,6-$(CH_3)_2$ | " | |
| 4 | 2,4,5-$(CH_3)_3$ | " | |
| 5 | 2-$CH_3$, 5-Cl | " | $n_D^{20}$ 1.5795 |
| 6 | 2-Cl, 6-$CH_3$ | " | Schmp. 143-146° C |
| 7 | 2-$CH_3$, 5-Br | " | $n_D^{50}$ 1.5855 |
| 8 | 2-Cl, 5-tert.-$C_4H_9$ | " | |
| 9 | 2-$OCH_3$, 5-$NO_2$ | " | |
| 10 | 3,4-$(OCH_3)_2$ | " | Schmp. 92-94° C |
| 11 | 3-O-$CH_2$-O-4 | " | |
| 12 | 2,5-$Cl_2$ | | |

| Verbindung Nr. | X, Y, Z | Q | Physikalische Konstante |
|---|---|---|---|
| 13 | 2,5-$Br_2$ | " | Schmp. 114-119° C |
| 14 | 2,4,5-$Cl_3$ | " | |
| 15 | 2,6-$(CH_3)_2$,3-$NO_2$ | " | Schmp. 99-101° C |
| 16 | 2-$CH_3$,5-Cl | 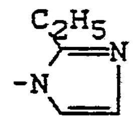 | |
| 17 | 2-$CH_3$,5-Br | " | |
| 18 | 2-$CH_3$,5-$NO_2$ | 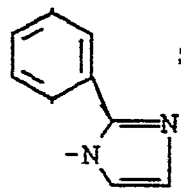 | Schmp. 145-150° C |
| 19 | 2-$CH_3$, 5-$NO_2$ | 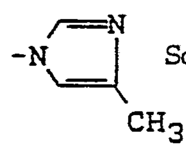 | Schmp. 111-115° C |
| 20 | 2-$CH_3$, 5-$NO_2$ | 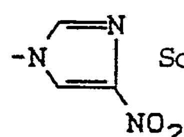 | Schmp. 150-155° C |
| 21 | 2-$CH_3$, 5-$NO_2$ | 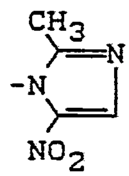 | Schmp. 180-190° C |
| 22 | 2-$CH_3$, 5-$NO_2$ | 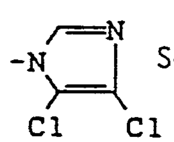 | Schmp. 125-130°C |

| Verbindung Nr. | X, Y, Z | Q | Physikalische Konstante |
|---|---|---|---|
| 23 | 2-$CH_3$, 5-Br | 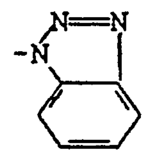 | Schmp. 121-127° C |
| 24 | 2-Cl, 6-$CH_3$ | " | Schmp. 120-122.5 °C |
| 25 | 2-$CH_3$, 5-Cl | " | Schmp. 112-117° C |
| 26 | 2-$OCH_3$, 5-Br | " | Schmp. 167-170° C |
| 27 | 2,5-$(CH_3)_2$ | 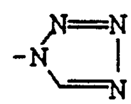 | Schmp. 111-116° C |
| 28 | 2-$CH_3$, 5-$NO_2$ | " | |
| 29 | 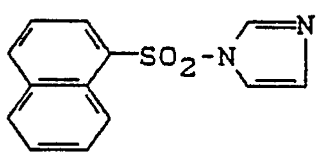 | | Schmp. 116-119° C |
| 30 | 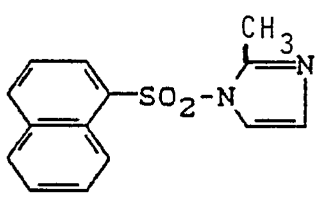 | | |
| 31 | 2-$CH_3$, 4-Br | 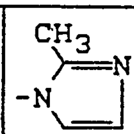 | |

## Verwendungsbeispiele

### Vergleichsverbindung A-1:

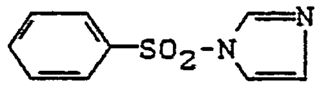

(bekannt aus der JP-OS 64181-1979).

### Vergleichsverbindung A-2:

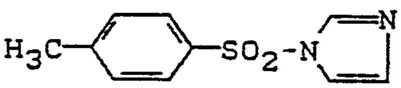

(bekannt aus der JP-OS 64181-1979).

### Vergleichsverbindung C-1:

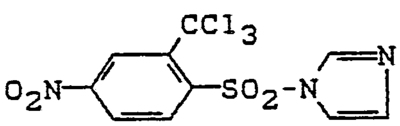

(bekannt aus der JP-OS 28053/1982).

### Beispiel A

Test der Behandlung von Tomaten-Braunfäule

Tomatenpflanzen (Varietät Kurihara), die in Porzellantöpfen mit einem Durchmesser von 9 cm gezogen worden waren, wurden durch Sprühen einer Emulsion einer aktiven Verbindung mit Hilfe einer Spritzpistole auf die Pflanzen behandelt.

Einen Tag nach dem Sprühen wurden die Pflanzen mit einer wäßrigen Sporen-Suspension des die Braunfäule verursachenden Organismus inokuliert. Die Pflanzen wurden in einem klimatisierten Raum bei konstanter Temperatur von 22°C und einer relativen Luftfeuchtigkeit von wenigstens 90 % eine Nacht stehen gelassen.

Nach 5 Tagen wurde die Infektion der Pflanzen durch die Messung der Fläche des Auftretens von Pusteln auf den Pflanzen und durch die Bewertung des Infektionsgrades mittels der folgenden Bewertungsskala bestimmt.

| Grad der Infektion | Fläche des Auftretens von Pusteln auf Pflanzen (%, bezogen auf die Gesamtfläche der Pflanzen) |
|---|---|
| 0 | 0 |
| 0,5 | bis zu 2 |
| 1 | 3 bis 5 |
| 2 | 6 bis 15 |
| 3 | 16 bis 30 |
| 4 | 31 bis 50 |
| 5 | 51 oder mehr |

Grad der Zerstörung (%)

$$= \frac{\left[\text{Grad der Infektion bei der Gruppe der unbehandelten Pflanzen}\right] - \left[\text{Grad der Infektion bei der Gruppe der behandelten Pflanzen}\right]}{\left[\text{Grad der Infektion bei der Gruppe der unbehandelten Pflanzen}\right]} \times 100$$

Die Ergebnisse der Tests, in denen eine Anzahl der vorliegenden aktiven Verbindungen eingestezt wurden, sind in Tabelle A aufgeführt.

## Tabelle A

| Aktive Verbindung Nr. | Konzentration der aktiven Verbindung (ppm) | Grad der Zerstörung (%) |
|---|---|---|
| 6 | 500 | 100 |
| 19 | " | 100 |
| 22 | " | 100 |
| 25 | " | 100 |
| 27 | " | 100 |
| 29 | " | 100 |
| Vergleichsverbindung | | |
| A-1 | 500 | 40 |
| A-2 | " | 50 |
| C-1 | " | 20 |

**Ansprüche**

1. Sulfonylazole der Formel (I)

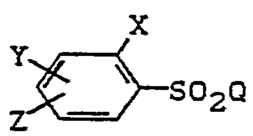

(I) ,

in der
X Wasserstoff, Alkyl, Halogen, Alkoxy oder Aryl bezeichnet,
Y Wasserstoff, Alkyl, Halogen, Nitro oder Alkoxy bezeichnet,
Z Wasserstoff, Alkyl, Halogen oder Alkoxy bezeichnet oder
Y und Z zusammen einen gegebenenfalls substituierten cyclischen Ring bezeichnen, der Hetero-Atome enthalten kann,
Q einen Rest der Formel

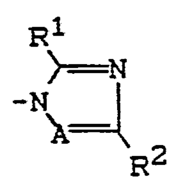

bezeichnet, in der
A einen Rest -N= oder einen Rest
$-\underset{R^3}{C}=$ bezeichnet,
und worin
$R^1$ Wasserstoff, Alkyl oder Aryl bezeichnet und

$R^2$ und $R^3$ jeweils Wasserstoff, Alkyl, Halogen oder Nitro bezeichnen,

mit der Maßgabe, daß nur in dem Fall, in dem Y und Z einen gegebenenfalls substituierten Ring bezeichnen, sämtliche R-Substituenten gleichzeitig Wasserstoff sein können, oder

Q einen 1H-Benzotriazol-1-yl-Rest oder 1H-Tetrazol-1-yl-Rest bezeichnet.

2. Sulfonylazole der Formel (I) nach Anspruch 1, dadurch gekennzeichnet, daß

X Alkyl mit 1 bis 4 Kohlenstoff-Atomen bezeichnet,

Y Fluor, Chlor, Brom, Nitro oder Alkyl mit 1 bis 4 Kohlenstoff-Atomen bezeichnet,

Z Wasserstoff bezeichnet und

Q einen Rest der Formel

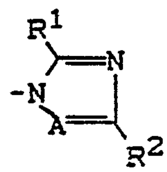

,

bezeichnet, in der

A einen Rest

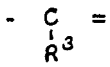 bezeichnet,

$R^1$ Wasserstoff oder Alkyl mit 1 bis 4 Kohlenstoff-Atomen bezeichnet und

$R^2$ und $R^3$ jeweils Wasserstoff oder Halogen bezeichnen,

mit der Maßgabe, daß nicht sämtliche R-Substituenten gleichzeitig Wasserstoff sind, oder

Q einen 1H-Benzotriazol-1-yl-Rest oder 1H-Tetrazol-1-yl-Rest bezeichnet.

3. Sulfonylazole der Formel (I) nach Anspruch 1, dadurch gekennzeichnet, daß

X Methyl bezeichnet,

Y Chlor, Brom, Nitro oder Alkyl mit 1 bis 3 Kohlenstoff-Atomen bezeichnet,

Z Wasserstoff bezeichnet und

Q einen 2-Methylimidazol-1-yl-Rest, einen 4,5-Dichloroimidazol-1-yl-Rest, einen 1H-Benzotriazol-1-yl-Rest oder einen 1H-Tetrazol-1-yl-Rest bezeichnet.

4. Sulfonylazole der Formel (I) nach Ansprüchen 1 bis 3, ausgewählt aus den nachstehenden Verbindungen:

1-(2-Chloro-6-methylbenzolsulfonyl)-2-methylimidazol der Formel

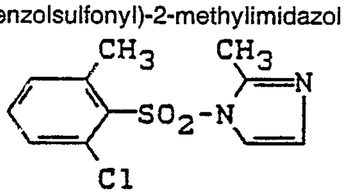

,

1-(2,5-Dimethylbenzolsulfonyl)-1H-tetrazol der Formel

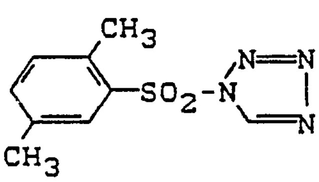

,

1-(2-Methyl-5-nitrobenzolsulfonyl)-4,5-dichloroimidazol der Formel

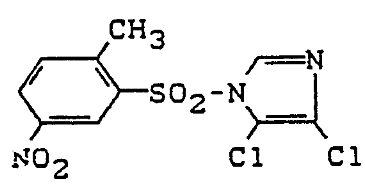

und 1-(5-Chloro-2-methyl-benzolsulfonyl)-1H-benzotriazol der Formel

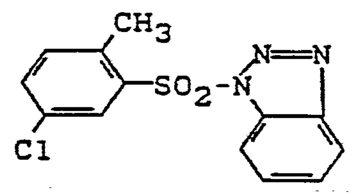

5. Verfahren zur Herstellung von Sulfonylazolen der Formel (I)

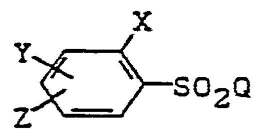

(I)

in der

X Wasserstoff, Alkyl, Halogen, Alkoxy oder Aryl bezeichnet,

Y Wasserstoff, Alkyl, Halogen, Nitro oder Alkoxy bezeichnet,

Z Wasserstoff, Alkyl, Halogen oder Alkoxy bezeichnet oder

Y und Z zusammen einen gegebenenfalls substituierten cyclischen Ring bezeichnen, der Hetero-Atome enthalten kann,

Q einen Rest der Formel

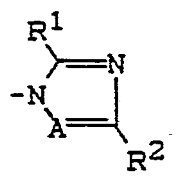

bezeichnet, in der

A einen Rest -N= oder einen Rest

$-\underset{R^3}{\underset{|}{C}}=$ bezeichnet,

und worin

$R^1$ Wasserstoff, Alkyl oder Aryl bezeichnet und

$R^2$ und $R^3$ jeweils Wasserstoff, Alkyl, Halogen oder Nitro bezeichnen,

mit der Maßgabe, daß nur in dem Fall, in dem Y und Z einen gegebenenfalls substituierten Ring bezeichnen, sämtliche R-Subsituenten gleichzeitig Wasserstoff sein können, oder

Q einen 1H-Benzotriazol-1-yl-Rest oder 1H-Tetrazol-1-yl-Rest bezeichnet,

dadurch gekennzeichnet, daß

eine Verbindung der Formel (II)

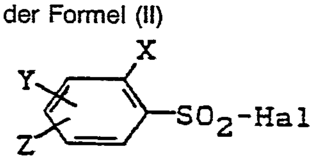

(II)

in der X, Y und Z die oben angegebenen Bedeutungen haven und Hal Halogen bezeichnet, mit einer Verbindung der Formel (III)

M -Q (III)

in der

Q die oben angegebenen Bedeutungen hat,

M Wasserstoff, ein Alkalimetall oder ein Erdalkalimetall-Äquivalent bezeichnet,

in Gegenwart eines inerten Lösungsmittels und gegebenenfalls in Gegenwart einer Base ungesetzt wird.

6. Fungizide Mittel für Landwirtschaft und Gartenbau, dadurch gekennzeichnet, daß sie wenigstens ein Sulfonylazol der Formel (I) gemäß den Ansprüchen 1 und 5 enthalten.

7. Verfahren zur Bekämpfung von Pflanzenkrankheiten, dadurch gekennzeichnet, daß man Sulfonylazole der Formel (I) gemäß den Ansprüchen 1 und 5 auf die Pathogene und/oder den Ort ihres Auftretens oder den Ort des Auftretens der Pflanzenkrankheit einwirken läßt.

8. Verwendung von Sulfonylazolen der Formel (I) gemäß den Ansprüchen 1 und 5 zur Bekämpfung von Pflanzenkrankheiten.

9. Verfahren zur Herstellung von funigziden Mitteln für Landwritschaft und Gartenbau, dadurch gekennzeichnet, daß Sulfonylazole der Formel (I) gemäß den Ansprüchen 1 und 5 mit Streckmitteln und/oder grenzflächenaktiven Mitteln vermischt werden.

Europäisches Patentamt

# EUROPÄISCHER RECHERCHENBERICHT

Nummer der Anmeldung

EP 87103384.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | CHEMICAL ABSTRACTS, Band 97, Nr. 25, 20. Dezember 1982, Columbus, Ohio, USA<br>SANKYO KAGAKU K.K. "8-Quinoline sulfonyl derivatives"<br>Seite 855, Spalte 2, Zusammenfassung-Nr. 216195t<br>& Jpn. Kokai Tokkyo Koho JP 82 116 067, 19 Juli 1982<br>-- | 1,5 | C 07 D 233/54<br>C 07 D 249/08<br>C 07 D 249/16<br>C 07 D 257/04<br>C 07 D 401/12<br>A 01 N 43/50<br>A 01 N 43/653<br>A 01 N 43/713 |
| X | EP - A2/A3 - 0 044 394 (BASF AKTIENGESELLSCHAFT)<br>* Ansprüche 1-7 *<br>-- | 1,5-9 | |
| X | EP - A2/A3 - 0 173 918 (NIHON TOKUSHU NOYAKU SEIZO K.K.)<br>* Ansprüche 1,4,5-9 *<br>-- | 1,5-9 | **RECHERCHIERTE SACHGEBIETE (Int. Cl 4)** |
| A | EP - A1 - 0 091 794 (ELI LILLY AND COMPANY)<br>* Anspruch 1 *<br>-- | 1,6-9 | C 07 D 233/00<br>C 07 D 249/00<br>C 07 D 257/00<br>C 07 D 401/00<br>A 01 N 43/00 |
| A | CHEMICAL ABSTRACTS, Band 97, Nr. 19, 8. November 1982, Columbus, Ohio, USA<br>FUJIMOTO PHARMACEUTICAL CO. "Tosylcimetidine"<br>Seite 719, Spalte 1, Zusammenfassung-Nr. 162986f<br>& Jpn. Kokai Tokkyo Koho JP 82 120 574, 27 Juli 1982<br>---- | 1,6-9 | |

Der vorliegende Recherchenbericht wurde für alle Patentansprüche erstellt.

| Recherchenort | Abschlußdatum der Recherche | Prüfer |
|---|---|---|
| WIEN | 26-06-1987 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82